# EUROPEAN PATENT APPLICATION

(11) **EP 4 553 143 A1**
(43) Date of publication of application: **14.05.2025**
(21) Application number: 23208978.9
(22) Date of filing: 10.11.2023
(51) Int. Cl.: C12M 1/34, G01N 27/416

(54) **MEASUREMENT SYSTEM AND METHOD FOR IN SITU CALIBRATION**

(71) Applicant: Imec VZW, 3001 Leuven (BE)
(72) Inventor: Henry, Olivier, 1950 Kraainem (BE)
(74) Representative: Körfer, Thomas

(57) **Abstract**

A measurement system (100) is provided for in situ calibration. The measurement system comprises a housing (101) comprising a culture medium (102) and at least one measurement probe (103) arranged in the culture medium (102), wherein the at least one measurement probe (103) comprises an opening (104), at least one sensor (105), and at least one channel (106) arranged from the opening (104) to the at least one sensor (105). The measurement system further comprises a calibration unit (108) connected to the opening (104), configured to supply a calibration fluid to the at least one sensor (105) via the at least one channel (106) .

## Description

The invention relates to in situ calibration of sensors, especially of Process Analytical Technology (PAT) sensor probes for bioprocess monitoring.

Generally, in drug bio-manufacturing, biological cells may be cultured under very controlled sterile conditions inside a bioreactor to maintain an optimal growth environment, where chemical and biochemical parameters may be monitored continuously, in situ. This can be achieved by using so-called "sensor probes" that may be placed inside the bioreactor. Furthermore, the sensors may be required to remain operational over the entire cell culture period, which may last somewhere between 3 and 6 weeks depending on the cell types.

However, current sensor technology cannot be operated for such an extended period without periodic calibration to guarantee the accuracy of the measurements. In addition, sensors cannot be taken out of the bioreactors to be calibrated, and re-inserted given the high risk of breaking sterility, i.e. introducing contamination into the cell culture.

In most settings, small samples may be collected from the reactor and may be analyzed off-line, and the results may be used to correct the in situ sensor readings. For example, the document US 8,117,924 B2 discloses an apparatus that may isolate a sample from a bulk fluid to measure its characteristics. However, this may require skilled personnel, and access to additional analytical facilities that may not necessarily be available immediately.

Accordingly, an object of the invention is to provide a measurement system and a method to facilitate in situ calibration of measurement sensors without needing to collect samples.

The object is solved by the features of the first independent claim for the measurement system and by the features of the second independent claim for the method. The dependent claims contain further developments.

According to a first aspect of the invention, a measurement system is provided for in situ calibration. The measurement system comprises a housing comprising a culture medium and at least one measurement probe arranged in the culture medium, wherein the at least one measurement probe comprises an opening, at least one sensor, and at least one channel arranged from the opening to the at least one sensor.

The measurement system further comprises a calibration unit connected to the opening, configured to supply a calibration fluid to the at least one sensor via the at least one channel. For instance, the calibration fluid may be sterilized before use. Advantageously, by means of the channel arranged in the measurement probe, the calibration unit may supply the calibration fluid to the sensor to perform in situ calibration without needing to collect samples of the culture medium.

Preferably, the at least one measurement probe comprises a membrane encompassing the at least one sensor, configured to confine the calibration fluid at or around the at least one sensor. Advantageously, a rapid diffusion and dilution of the calibration fluid into the culture medium may be prevented. Furthermore, the calibration accuracy may be improved, e.g., by confining the calibration fluid above the sensor during the calibration measurement.

Preferably, the calibration unit comprises a controller configured to control a flow of the calibration fluid in the at least one channel. In this regard, the flow can be bidirectional, i.e., from the calibration unit to the sensor as well as from the sensor to the calibration unit, especially within the channel.

Preferably, the controller is further configured to control a flow of the culture medium to the at least one sensor through the membrane. In this regard, the flow can be bidirectional, i.e., from the housing to the sensor as well as from the sensor to the housing, especially through the membrane.

Advantageously, the membrane may protect the sensors from the culture medium. Furthermore, by controlling the flow of the culture medium through the membrane, the effect of flow during the measurement and/or calibration can be prevented or limited.

Preferably, the calibration fluid comprises a calibration solution with a defined concentration. For example, the calibration fluid may comprise a calibration solution for a specific sensor, and the calibration unit may comprise such calibration fluids for respective sensors.

Preferably, the calibration fluid comprises a plurality of calibration solutions with different concentrations. For example, the calibration fluid may comprise a combination of calibration solutions for calibrating multiple sensors, and the calibration unit may comprise such combined calibration solutions of different concentrations.

Preferably, the membrane comprises a plurality of pores with an internal width from 1 micrometer to 5 micrometers. More preferably, the membrane may comprise a plurality of pores with an internal width from 10nm to 100nm. Advantageously, the pores may allow sample diffusion, e.g., proteins, at the sensor in a controlled manner, while preventing any contamination at the sensor surface.

Preferably, the calibration unit is further configured to supply a cleaning fluid to the at least one sensor via the at least one channel. Advantageously, measurement and/or calibration accuracy can be further improved.

Preferably, the at least one measurement probe, the calibration fluid, and the cleaning fluid are sterile. Advantageously, the sterile conditions of the bioprocess monitoring can be maintained.

Preferably, the at least one measurement probe further comprises at least one further sensor configured to be in contact with the culture medium. Advantageously, by means of the further sensor in direct contact with the culture medium, i.e. external or not confined by the membrane, a differential measurement can be performed, which may further improve the measurement and/or calibration accuracy.

Preferably, the at least one sensor and/or the at least one further sensor are electrochemical sensors. Alternatively, the at least one sensor and/or the at least one further sensor are optical sensors. Further alternatively, the at least one sensor and/or the at least one further sensor are mass-sensitive sensors. Further alternatively, the at least one sensor and/or the at least one further sensor are thermo-sensitive sensors.

Additionally or alternatively, the at least one sensor and/or the at least one further sensor are configured to measure at least one of a physical parameter, a chemical parameter, and a biochemical parameter of the culture medium. The parameters can be defined as but not limited to temperature, pH level, dissolved oxygen, glucose, lactate, glutamine and glutamate concentrations, ion concentration (e.g. sodium, calcium, magnesium) cell density, cell viability, electrical conductivity, oxidation reduction potential, and proteins concentrations.

Preferably, the housing is a cell culture vessel, preferably a bioreactor vessel. Alternatively, the housing may correspond to a sterile environment for environmental and/or industrial monitoring.

According to a second aspect of the invention, a method is provided for in situ calibration. The method comprises the steps of providing a housing comprising a culture medium; arranging at least one measurement probe comprising an opening, at least one sensor, and at least one channel arranged from the opening to the at least one sensor in the culture medium; connecting a calibration unit to the opening; and supplying, by the calibration unit, a calibration fluid to the at least one sensor via the at least one channel.

Preferably, the method further comprises the step of providing a membrane encompassing the at least one sensor to confine the calibration fluid at or around the at least one sensor.

Preferably, the calibration unit comprises a controller, and the method further comprises the step of controlling, via the controller, a flow of the calibration fluid in the at least one channel. Additionally or alternatively, the method further comprises the step of controlling, via the controller, a flow of the culture medium to the at least one sensor through the membrane.

It is to be noted that the method according to the second aspect corresponds to the measurement system according to the first aspect and its implementation forms. Accordingly, the method of the second aspect may have corresponding implementation forms. Further, the method of the second aspect achieves the same advantages and effects as the measurement system of the first aspect and its respective implementation forms.

Exemplary embodiments of the invention are now further explained with respect to the drawings by way of example only, and not for limitation. In the drawings:
- Fig. 1: shows a first exemplary embodiment of the measurement system according to the first aspect of the invention;
- Fig. 2: shows an exemplary embodiment of the calibration unit;
- Fig. 3: shows an exemplary in situ calibration operation;
- Fig. 4: shows a second exemplary embodiment of the measurement system according to the first aspect of the invention; and
- Fig. 5: shows an exemplary embodiment of the method according to the second aspect of the invention.

Reference will now be made in detail to the embodiments of the present invention, examples of which are illustrated in the accompanying drawings. However, the following embodiments of the present invention may be variously modified and the range of the present invention is not limited by the following embodiments.

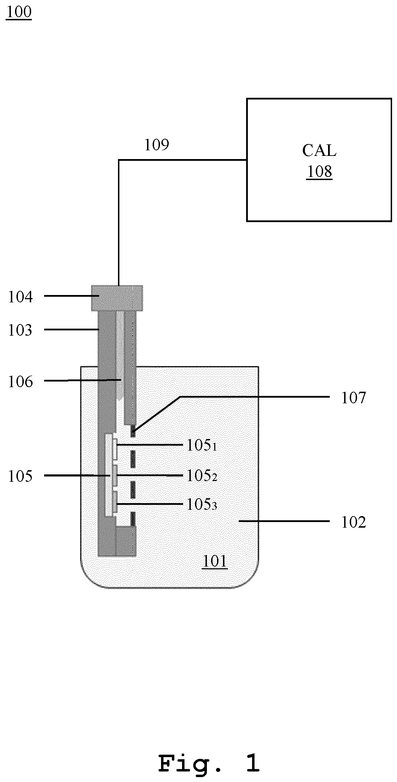

In Fig. 1, a first exemplary embodiment of the measurement system 100 according to the first aspect of the invention is illustrated. The measurement system 100 may comprise a housing 101, e.g., a bioreactor vessel, comprising a cell-culture medium 102 and a measurement probe 103 arranged in the cell-culture medium 102, especially inside of the housing 101.

The measurement probe 103 may comprise an opening 104, an array of sensors 105, herein exemplarily shown with three sensors 105₁, 105₂, 105₃, and a channel 106 arranged from the opening 104 to the array of sensors 105, especially within the measurement probe 103.

The measurement probe 103 may further comprise a membrane 107 encompassing the array of sensors 105, especially to protect the array of sensors 105 from the cell-culture medium 102. For instance, the membrane 107 may be arranged, i.e., the position of the membrane 107 may be aligned, with respect to the position/location of the array of sensors 105 in the measurement probe 103.

For example, the probe material, especially of the body of the measurement probe 103, may differ from the membrane material. In this regard, the measurement probe 103 may comprise a slot or an aperture, especially on the body of the measurement probe 103, corresponding to the position/location of the array of sensors 105, and the membrane 107 may be attached to the measurement probe 103, especially to the body of the measurement probe 103, at the slot or the aperture, especially covering the slot or the aperture.

The measurement system 100 may further comprise a calibration unit 108 connected to the opening 104 of the measurement probe 103 and further to the channel 106 inside of the measurement probe 103, for example via a flow path 109. The calibration unit 108 may supply a calibration fluid to the array of sensors 105 through the channel 106, e.g., via the flow path 109 through the opening 104 of the measurement probe 103.

In this regard, the membrane 107 may confine the calibration fluid above the array of sensors 105, especially during the calibration measurement, and may prevent rapid diffusion and dilution of the calibration fluid into the housing 101.

For example, the membrane 107 may comprise pores having an internal width sufficient to pass molecules, such as proteins, through the membrane 107. For instance, the pores may have an internal width of about 1 micrometer to 5 micrometers, preferably an internal width of about 10nm to 100nm, especially to allow sample diffusion to the array of sensors 105 while preventing cells contaminate the array of sensors 105, especially the surface of the sensors.

In other words, the membrane 107 may correspond to or be a one-way flow membrane, which may restrict the flow of the calibration fluid from the channel 106 and/or the array of sensors 105 of the measurement probe 103 into the housing 101. However, the membrane 107 may allow sample diffusion, such as protein molecules, to the array of sensors 105, especially by allowing the sample to flow from the housing 101, e.g., through the pores, into the measurement probe 103.

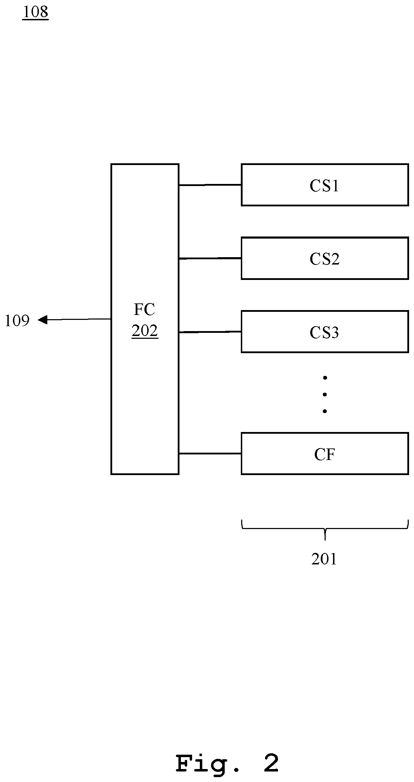

In Fig. 2, an exemplary embodiment of the calibration unit 108 is illustrated. The calibration unit 108 may comprise a storage section 201 and a flow controller 202 connected to the storage section 201. In this regard, the storage section 201 may act as a reservoir to store one or more calibration fluids or solutions CS1, CS2, CS3 and cleaning fluids CF.

For example, the calibration fluids CS1, CS2, CS3 may respectively correspond to the sensors 105₁, 105₂, 105₃ of the array of sensors 105, e.g., for a respective calibration specie. Alternatively, the calibration fluids CS1, CS2, CS3 may comprise different concentration of a mixture of calibration specie.

The flow controller 202 may comprise flow controlling means, such as a set of valves and a pump, that may be actuated on demand, e.g., via a software interface. As such, the flow controller 202 may push the calibration fluids CS1, CS2, CS3 and/or the cleaning fluid CF through the channel 106 to the array of sensors 105, e.g., via the flow path 109 through the opening 104 of the measurement probe 103.

Furthermore, the flow controller 202 may pull a sample from the housing 101 through the membrane 107 onto the array of sensors 105, especially to control the flow during the measurement.

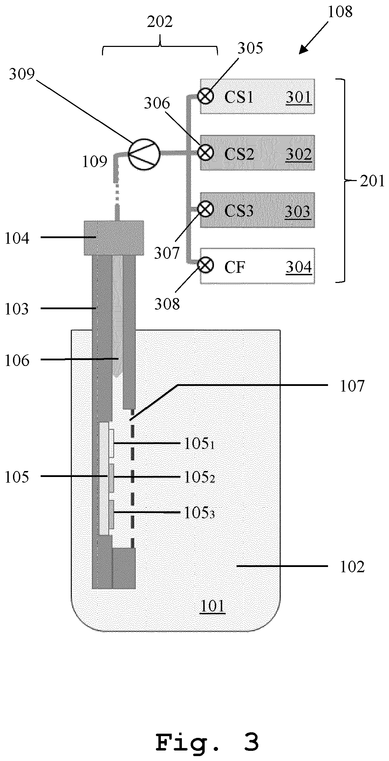

In Fig. 3, an exemplary in situ calibration operation of the measurement system 100 is illustrated. For example, the array of sensors 105 may comprise three sensors 105₁, 105₂, 105₃; each may measure or monitor a respective single physical or chemical or biochemical parameter.

The calibration unit 108, especially the storage section 201, may comprise a first calibration solution storage 301 containing a first calibration solution CS1 corresponding to the sensor 105₁, a second calibration solution storage 302 containing a second calibration solution CS2 corresponding to the sensor 105₂, a third calibration solution storage 303 containing a third calibration solution CS3 corresponding to the sensor 105₃, and a fourth storage 304 containing the cleaning fluid CF. The calibration solutions CS1, CS2, CS3 and the cleaning fluid CF may be sterile.

The flow controller 202 may comprise a first valve 305, e.g., a directional valve, connected to the first calibration solution storage 301 to control a flow of the first calibration solution CS1, a second valve 306 connected to the second calibration solution storage 302 to control a flow of the second calibration solution CS2, a third valve 307 connected to the third calibration solution storage 303 to control a flow of the third calibration solution CS3, and a fourth valve 308 connected to the fourth storage 304 to control a flow of the cleaning fluid CF.

The flow controller 202 may further comprise a pump 309 connected to the first valve 305, the second valve 306, the third valve 307, and the fourth valve 308, and further to the flow path 109. In this regard, the pump 309 may push one or more of the calibration solutions CS1, CS2, CS3, or the cleaning fluid CF to the sensors 105₁, 105₂, 105₃, via the channel 106 through the opening 104 of the measurement probe 103. The first valve 305, the second valve 306, the third valve 307, the fourth valve 308, and the pump 309 may be actuated on demand, e.g., via a software interface.

For instance, if a calibration measurement for the sensor 105₁ is needed, the first valve 305 may be operated or activated to enable a flow of the first calibration solution CS1, and the pump 309 may push the first calibration solution CS1 via the flow path 109 through the opening 104 and further through the channel 106 to the sensor 105₁.

Analogously, if the sensor 105₁ is required to be cleaned, the fourth valve 308 may be operated or activated to enable a flow of the cleaning fluid CF, and the pump 309 may push the cleaning fluid CF via the flow path 109 through the opening 104 and further through the channel 106 to the sensor 105₁.

For instance, is case of a parameter measurement, the pump 309 may pull a sample from the housing through the membrane 107 onto the sensors 105₁, 105₂, 105₃. In this regard, the membrane 107 may comprise a uniform surface or may comprise different surfaces corresponding to the respective sensors 105₁, 105₁, 105₃, e.g., with different pore sizes, especially to allow selective measurements.

It is to be noted that the opening 104 of the measurement probe 103 may additionally act as a sealing around the flow path 109 connecting the channel 106 within the measurement probe 103. Since the measurement probe 103, the calibration solutions CS1, CS2, CS3, and the cleaning fluid CF may be sterilized before use, the in situ calibration may not affect the sterility of the measurement probe 103 and/or the housing 101.

Moreover, since the first valve 305, the second valve 306, the third valve 307, the fourth valve 308, and the pump 309 may be actuated via a software interface, in situ automated calibration of the measurement probe 103, especially of the array of sensors 105, can be achieved without needing to collect samples.

For example, especially for calibrating the sensors 105₁, 105₂, 105₃, respective calibration solutions CS1, CS2, CS3 may be fed to the sensors 105₁, 105₂, 105₃, which correspond to a desired or preset value or data of the sensors 105₁, 105₂, 105₃. Using the respective calibration solutions CS1, CS2, CS3, one or more measurement values or data may be collected from the sensors 105₁, 105₂, 105₃, and may be compared with the desired or preset value or data.

Afterwards, an adjustment or a set of adjustments may be performed to ensure the measurement accuracy of the sensors 105₁, 105₂, 105₃.

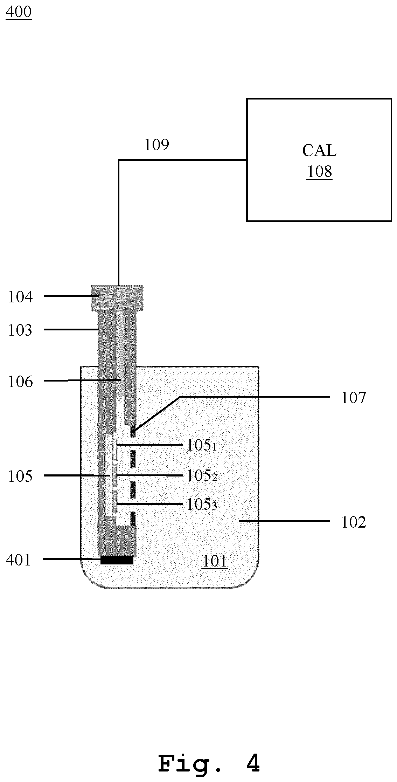

In Fig. 4, a second exemplary embodiment of the measurement system 400 according to the first aspect of the invention is illustrated. The measurement system 400 differs from the measurement system 100 in that the measurement probe 103 of the measurement system 400 may additionally comprise a further sensor or an additional array of sensors 401, especially arranged at the measurement probe 103 to be in direct contact with the cell-culture medium 102.

For example, the further sensor or the additional array of sensors 401 may be arranged at the measurement probe 103 outside of the membrane 107. In other words, the membrane 107 may not encompass the further sensor or the additional array of sensors 401, especially to facilitate differential measurements.

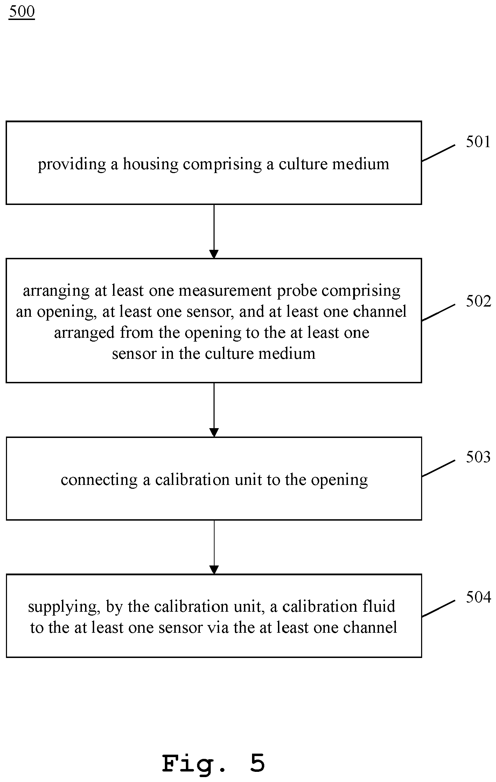

In Fig. 5, an exemplary embodiment of the method 500 according to the second aspect of the invention is illustrated. In a first step 501, a housing comprising a culture medium is provided. In a second step 502, at least one measurement probe comprising an opening, at least one sensor, and at least one channel arranged from the opening to the at least one sensor is arranged in the culture medium. In a third step 503, a calibration unit is connected to the opening of the at least one measurement probe. In a fourth step 504, a calibration fluid is supplied by the calibration unit to the at least one sensor via the at least one channel.

It is important to note that, in the description as well as in the claims, the word "comprising" does not exclude other elements or steps and the indefinite article "a" or "an" does not exclude a plurality. A single element or other unit may fulfill the functions of several entities or items recited in the claims. Furthermore, the word "connected" implies that the elements may be directly connected together or may be coupled through one or more intervening elements. Moreover, the disclosure with regard to any of the aspects is also relevant with regard to the other aspects of the disclosure.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

## Claims

1. A measurement system (100, 400) for in situ calibration, comprising:
a housing (101) comprising a culture medium (102) and at least one measurement probe (103) arranged in the culture medium (102), wherein the at least one measurement probe (103) comprises an opening (104), at least one sensor (105), and at least one channel (106) arranged from the opening (104) to the at least one sensor (105); and
a calibration unit (108) connected to the opening (104), configured to supply a calibration fluid to the at least one sensor (105) via the at least one channel (106).

2. The measurement system according to claim 1,
wherein the at least one measurement probe (103) comprises a membrane (107) encompassing the at least one sensor (105), configured to confine the calibration fluid at or around the at least one sensor (105).

3. The measurement system according to claim 1 or 2,
wherein the calibration unit (108) comprises a controller (202) configured to control a flow of the calibration fluid in the at least one channel (106).

4. The measurement system according to claim 3,
wherein the controller (202) is further configured to control a flow of the culture medium (102) to the at least one sensor (105) through the membrane (107).

5. The measurement system according to any of claims 1 to 4,
wherein the calibration fluid comprises a calibration solution with a defined concentration.

6. The measurement system according to any of claims 1 to 4,
wherein the calibration fluid comprises a plurality of calibration solutions with different concentrations.

7. The measurement system according to claim 2,
wherein the membrane (107) comprises a plurality of pores with an internal width from 1 micrometer to 5 micrometers, preferably from 10nm to 100nm.

8. The measurement system according to any of claims 1 to 7,
wherein the calibration unit (108) is further configured to supply a cleaning fluid to the at least one sensor (105) via the at least one channel (106).

9. The measurement system according to claim 8,
wherein the at least one measurement probe (103), the calibration fluid, and the cleaning fluid are sterile.

10. The measurement system according to any of claims 1 to 9,
wherein the at least one measurement probe (103) further comprises at least one further sensor (401) configured to be in contact with the culture medium (102).

11. The measurement system according to claim 10,
wherein the at least one sensor (105) and/or the at least one further sensor (401) are electrochemical sensors, and/or
wherein the at least one sensor (105) and/or the at least one further sensor (401) are configured to measure at least one of a physical parameter, a chemical parameter, and a biochemical parameter of the culture medium (102).

12. The measurement system according to any of claims 1 to 11,
wherein the housing (101) is a cell culture vessel, preferably a bioreactor vessel.

13. A method (500) for in situ calibration, comprising:
providing (501) a housing comprising a culture medium;
arranging (502) at least one measurement probe comprising an opening, at least one sensor, and at least one channel arranged from the opening to the at least one sensor in the culture medium;
connecting (503) a calibration unit to the opening; and
supplying (504), by the calibration unit, a calibration fluid to the at least one sensor via the at least one channel.

14. The method according to claim 13,
wherein the method further comprising providing a membrane encompassing the at least one sensor to confine the calibration fluid at or around the at least one sensor.

15. The method according to claim 13 or 14,
wherein the calibration unit comprises a controller, and the method further comprising:
controlling, via the controller, a flow of the calibration fluid in the at least one channel; and/or
controlling, via the controller, a flow of the culture medium to the at least one sensor through the membrane.
